(19)

Europäisches Patentamt
European Patent Office
Office européen des brevets

(11) **EP 3 826 022 A1**

(12) **EUROPEAN PATENT APPLICATION**

(43) Date of publication:
**26.05.2021 Bulletin 2021/21**

(51) Int Cl.:
**G16C 20/40** (2019.01)     **G16B 15/00** (2019.01)

(21) Application number: **20202456.8**

(22) Date of filing: **18.10.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **22.11.2019   JP 2019211007**

(71) Applicant: **FUJITSU LIMITED**
**Kanagawa 211-8588 (JP)**

(72) Inventor: **SATO, Hiroyuki**
**Kanagawa, 211-8588 (JP)**

(74) Representative: **Haseltine Lake Kempner LLP**
**Cheapside House**
**138 Cheapside**
**London**
**EC2V 6BJ (GB)**

(54)    **STRUCTURE SEARCH METHOD, STRUCTURE SEARCH APPARATUS, AND PROGRAM**

(57)    A structure search method includes: sequentially arranging, by a computer, n compound groups at each lattice point, of a plurality of lattice points, of a three-dimensional lattice space to create a three-dimensional structure of a compound in the three-dimensional lattice space, the n compound groups being coupled to each other in the compound; and calculating a minimum energy of an Ising model by performing a ground state search using an annealing method on the Ising model transformed based on constraint conditions for each of the lattice points, the constraint conditions including: a first constraint that each of the n compound groups is arranged at only one lattice point; a second constraint that the n compound groups do not overlap with each other at each of the lattice points; and a third constraint that relates to coupling of the n compound groups and increases energy of the Ising model calculated when the constraint is not satisfied.

## FIG. 12

S101 — DEFINE THREE-DIMENSIONAL LATTICE SPACE

S102 — SET A SET OF LATTICE POINTS OF DESTINATION OF i-th AMINO ACID RESIDUE AS $V_i$

S103 — ALLOCATE SPATIAL INFORMATION TO $X_1$ TO $X_n$

S104 — CREATE ISING MODEL

S105 — OBTAIN MINIMUM VALUE OF E(x) BY ANNEALING MACHINE

S106 — OUTPUT OF RESULT

EP 3 826 022 A1

**Description**

FIELD

The embodiments discussed herein are related to a structure search method, a structure search apparatus, and a program.

BACKGROUND

In recent years, in a scene such as a drug discovery, it may be unavoidable to obtain a stable structure of a molecule having a large size by using an information processing apparatus (computer). However, for example, in a case of a large size molecule such as a protein, it may be difficult to search for a stable structure within a practical time in a calculation under careful consideration of all atoms.

Therefore, a technique for reducing the calculation time by roughly capturing the structure of a molecule (coarse-graining) has been studied.

As a technique for coarse-graining of a molecular structure, for example, a technique has been studied in which a molecular structure is subjected to coarse-graining into a linear (one series) simple cubic lattice structure based on one-dimensional sequence information of an amino acid residue in a protein and is treated as a lattice protein. There has been reported a technique for searching for a stable structure at high speed by using the technique of quantum annealing in a lattice protein.

In such a technique of searching for a stable structure in a lattice protein using an annealing machine, it is hard to simultaneously satisfy a plurality of constraint conditions, and it is difficult to efficiently search for a stable structure.

Related techniques are disclosed in, for example, R. Babbush et.al., Construction of Energy Functions for Lattice Heteropolymer Models: A Case Study in Constraint Satisfaction Programming and Adiabatic Quantum Optimization, Advances in Chemical Physics, 155, 201-244, April 04, 2014.

In one aspect, it is desirable to provide a structure search method, a structure search program, and a structure search apparatus capable of efficiently searching for a stable structure.

SUMMARY

According to an aspect of the embodiments, a structure search method includes: sequentially arranging, by a computer, n compound groups at each lattice point, of a plurality of lattice points, of a three-dimensional lattice space to create a three-dimensional structure of a compound in the three-dimensional lattice space, the n compound groups being coupled to each other in the compound; and calculating a minimum energy of an Ising model by performing a ground state search using an annealing method on the Ising model transformed based on constraint conditions for each of the lattice points, the constraint conditions including: a first constraint that each of the n compound groups is arranged at only one lattice point; a second constraint that the n compound groups do not overlap with each other at each of the lattice points; and a third constraint that relates to coupling of the n compound groups and increases energy of the Ising model calculated when the constraint is not satisfied.

In one aspect of the embodiment, it is possible to provide a structure search method, a structure search program, and a structure search apparatus that search for a stable structure.

BRIEF DESCRIPTION OF DRAWINGS

FIG. 1A is a schematic diagram illustrating an example in which a protein is coarse-grained to search for a stable structure (part 1).
FIG. 1B is a schematic diagram illustrating an example in which a protein is coarse-grained to search for a stable structure (part 2).
FIG. 1C is a schematic diagram illustrating an example in which a protein is coarse-grained to search for a stable structure (part 3).
FIG. 2A is a schematic diagram for explaining an example of a diamond encoding method (part 1).
FIG. 2B is a schematic diagram for explaining an example of the diamond encoding method (part 2).
FIG. 2C is a schematic diagram for explaining an example of the diamond encoding method (part 3).
FIG. 2D is a schematic diagram for explaining an example of the diamond encoding method (part 4).
FIG. 2E is a schematic diagram for explaining an example of the diamond encoding method (part 5).
FIG. 3 is a diagram for explaining an example of $H_{one}$.

FIG. 4 is a diagram for explaining an example of $H_{olap}$.

FIG. 5 is a diagram for explaining an example of $H_{conn}$.

FIG. 6 is a diagram for explaining an example of $H_{pair}$.

FIG. 7 is a diagram for explaining an example of a third constraint.

FIG. 8 illustrates a block diagram of an example of a structure search apparatus disclosed herein.

FIG. 9 is a diagram illustrating a configuration example of the structure search apparatus disclosed herein.

FIG. 10 is a diagram illustrating another configuration example of the structure search apparatus disclosed herein.

FIG. 11 is a diagram illustrating another configuration example of the structure search apparatus disclosed herein.

FIG. 12 is a flowchart illustrating an example of a method for searching for a stable structure of a protein.

FIG. 13 is a diagram illustrating a case where each lattice having a radius r is denoted by $S_r$.

FIG. 14A is a diagram illustrating a set of lattice points of a destination of an amino acid residue (part 1).

FIG. 14B is a diagram illustrating a set of lattice points of a destination of an amino acid residue (part 2).

FIG. 14C is a diagram illustrating a set of lattice points of a destination of an amino acid residue (part 3).

FIG. 14D is a diagram illustrating a set of lattice points of a destination of an amino acid residue (part 4).

FIG. 15 is a diagram illustrating $S_1$, $S_2$, $S_3$ in three dimensions.

FIG. 16A is a diagram illustrating an example of a state in which spatial information is allocated to each of bits $X_1$ to $X_n$ (part 1).

FIG. 16B is a diagram illustrating an example of a state in which spatial information is allocated to each of the bits $X_1$ to $X_n$ (part 2).

FIG. 16C is a diagram illustrating an example of a state in which spatial information is allocated to each of the bits $X_1$ to $X_n$ (part 3).

FIG. 17 is a diagram for explaining $H_{one}$.

FIG. 18 is a diagram for explaining $H_{olap}$.

FIG. 19A is a diagram for explaining $H_{pair}$ (part 1).

FIG. 19B is a diagram for explaining $H_{pair}$ (part 2).

FIG. 20 is a diagram illustrating an example of a weight file.

FIG. 21 is a diagram illustrating an example of a functional configuration of an optimization apparatus (control unit) used in an annealing method.

FIG. 22 is a block diagram illustrating an example of a circuit level of a transition control unit.

FIG. 23 is a diagram illustrating an example of an operation flow of the transition control unit.

FIG. 24 is a data configuration example of a storage unit of the structure search apparatus.

FIG. 25 is a processing flow corresponding to the data configuration example of FIG. 24.

DESCRIPTION OF EMBODIMENTS

First, a method for obtaining a folding structure of a protein by the diamond encoding method, which is one of techniques using a lattice protein, will be described before describing the details of the technique disclosed herein.

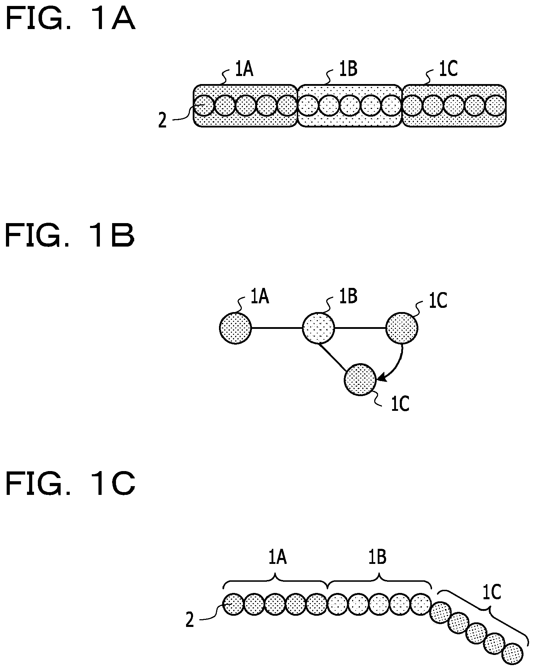

When searching for a structure of the protein (or peptide) using the lattice protein, first, the protein is coarse-grained. As illustrated in FIG. 1A, for example, the coarse-graining of the protein is performed by creating a coarse-grained model by coarse-graining of atoms 2 constituting the protein into coarse-grained particles 1A, 1B, and 1C, each of which is a unit for each amino acid residue.

Next, the created coarse-grained model is used to search for a stable binding structure. FIG. 1B illustrates an example of a case where the binding structure in which the coarse-grained particle 1C is located at an end point of an arrow is stable. The stable binding structure is searched for by the diamond encoding method described later.

As illustrated in FIG. 1C, the coarse-grained model is returned to an all-atoms model based on the stable binding structure searched for by using the diamond encoding method.

The diamond encoding method is a method of fitting particles (coarse-grained model) subjected to coarse-graining on a chain amino acid forming a protein to lattice points of a diamond lattice, and it is possible to express a three-dimensional protein structure.

In the following description, for simplification of explanation, the diamond encoding method used for a two-dimensional case will be described as an example.

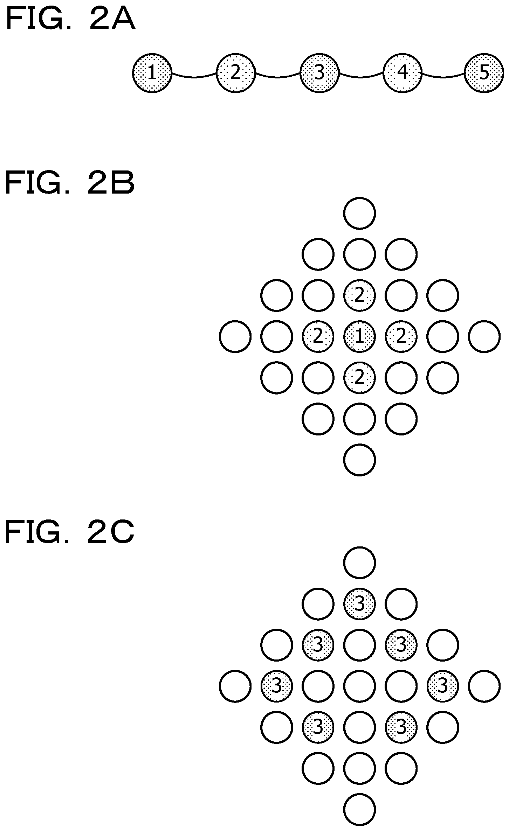

FIG. 2A illustrates an example of a structure in which a linear pentapeptide having five amino acid residues bound to each other has a linear structure. In FIG. 2A to FIG. 2E, numbers in circles represent a number of an amino acid residue in the linear pentapeptide.

In the diamond encoding method, first, when an amino acid residue having the number 1 is arranged at a center of a diamond lattice, as illustrated in FIG. 2B, a place where an amino acid residue having the number 2 may be arranged is limited to places adjacent to the center (places where the number 2 is given).

Subsequently, a place where an amino acid residue having the number 3 to be bound to the amino acid residue having the number 2 may be arranged is limited to places adjacent to the places where the number 2 is given in FIG. 2B (places where the number 3 is given in FIG. 2C).

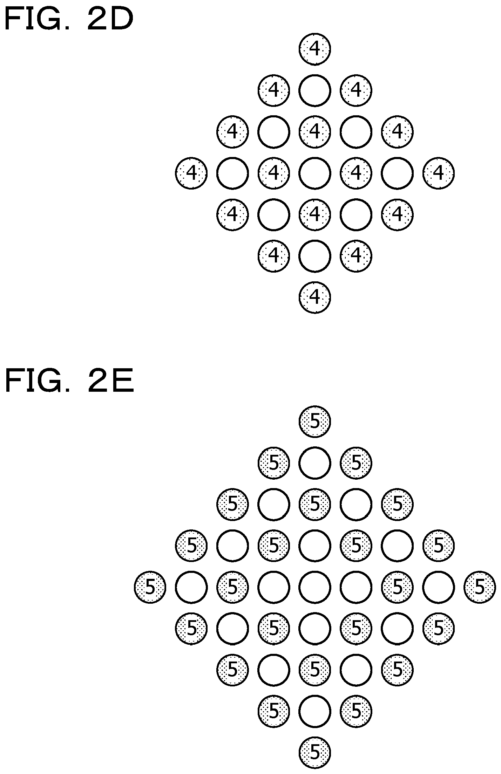

A place where an amino acid residue having the number 4 to be bound to the amino acid residue having the number 3 may be arranged is limited to places adjacent to the places where the number 3 is given in FIG. 2C (places where the number 4 is given in FIG. 2D).

A place where an amino acid residue having the number 5 to be bound to the amino acid residue having the number 4 may be arranged is limited to places adjacent to the places where the number 4 is given in FIG. 2D (places where the number 5 is given in FIG. 2E).

It is possible to express a coarse-grained structure of the protein by linking the places specified thus as arrangeable places in the order of the number of the amino acid residue.

Minimum energy of an Ising model is calculated by performing a ground state search using an annealing method on the Ising model transformed based on a constraint condition with respect to the coarse-grained structure of the protein. By doing so, it is possible to obtain the stable structure of the protein.

When $H_{one}$, $H_{olap}$, and $H_{conn}$ are set as the constraint conditions and $H_{pair}$ is set as a cost function, it is possible to express the total energy in the diamond encoding method as follows.

$$E(x) = H = H_{one} + H_{olap} + H_{conn} + H_{pair}$$

$H_{one}$ represents a constraint that the protein includes only one instance of each of the first to n-th amino acid residues.

$H_{olap}$ represents a constraint that the first to n-th amino acid residues do not overlap with each other.

$H_{conn}$ represents a constraint that the first to n-th amino acid residues are linked to each other.

$H_{pair}$ represents a cost function expressing an interaction between amino acid residues.

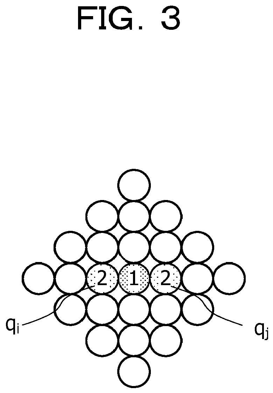

Regarding Hamiltonian ($H_{one}$), in a case where there are two amino acid residues as illustrated in FIG. 3, the Hamiltonian ($H_{one}$) represented by the following Formula (A) is positive. In other words, in the case where there are two amino acid residues, the Hamiltonian ($H_{one}$) according to this constraint condition increases the total energy.

$$H_{one} += C_1 q_i q_j \qquad \text{Formula (A)}$$

$C_1$ is a coefficient for weighting and is a positive integer. $q_i$ takes "1" or "0". $q_j$ takes "1" or "0".

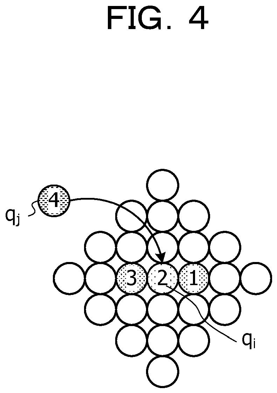

Regarding Hamiltonian ($H_{olap}$), in a case where certain amino acid residues overlap at a certain lattice point as illustrated in FIG. 4, the Hamiltonian ($H_{olap}$) represented by the following Formula (B) is positive. In other words, in a case where the certain amino acid residues overlap at a certain lattice point, the Hamiltonian ($H_{olap}$) according to this constraint condition increases the total energy.

$$H_{olap} += C_2 q_i q_j \qquad \text{Formula (B)}$$

$C_2$ is a coefficient for weighting and is a positive integer. $q_i$ takes "1" or "0". $q_j$ takes "1" or "0".

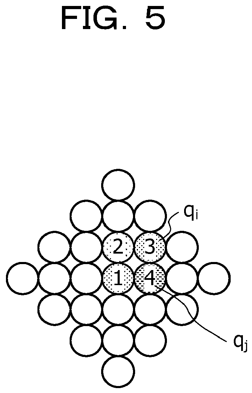

Regarding Hamiltonian ($H_{conn}$), in a case where two adjacent amino acid residues are linked as illustrated in FIG. 5, the Hamiltonian ($H_{conn}$) represented by the following Formula (C) is negative. In other words, in the case where two adjacent amino acid residues are linked, the Hamiltonian ($H_{conn}$) according to this constraint condition reduces the total energy.

$$H_{conn} -= C_3 q_i q_j \qquad \text{Formula (C)}$$

$C_3$ is a coefficient for weighting and is a positive integer. $q_i$ takes "1" or "0". $q_j$ takes "1" or "0".

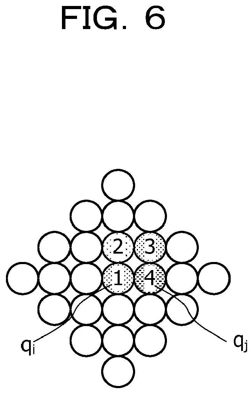

Regarding Hamiltonian ($H_{pair}$), in a case where two adjacent amino acid residues interact with each other as illustrated in FIG. 6, the Hamiltonian ($H_{pair}$) represented by the following Formula (D) is positive. In other words, in the case where two adjacent amino acid residues interact with each other, the Hamiltonian ($H_{olap}$) according to this constraint condition increases the total energy.

$$H_{\mathrm{pair}} \mathrel{+}= E_{14} q_i q_j$$

Formula (D)

$E_{14}$ is a coefficient relating to interaction and is a positive integer. $q_i$ takes "1" or "0". $q_j$ takes "1" or "0". The interaction is determined by a combination of two amino acid residues, and the interaction is determined with reference to, for example, the Miyazawa-Jernigan (MJ) matrix.

$H_{\mathrm{one}}$ and $H_{\mathrm{olap}}$ increase the total energy when the respective constraints are not satisfied. In other words, $H_{\mathrm{one}}$ and $H_{\mathrm{olap}}$ are constraints that the structure of a protein is destabilized when the respective constraints are not satisfied.

Whereas, normally, $H_{\mathrm{conn}}$ reduces the total energy when the constraint is not satisfied. In other words, $H_{\mathrm{conn}}$ is a constraint that the structure of a protein is stabilized when the constraint is not satisfied.

Therefore, the relationship between $H_{\mathrm{one}}$ and $H_{\mathrm{olap}}$, and $H_{\mathrm{conn}}$ is non-independent, and when one constraint is satisfied, another constraint is less likely to be satisfied. As a result, it is difficult to efficiently search for a stable structure.

Therefore, with the disclosed technique, the relationship between $H_{\mathrm{one}}$ and $H_{\mathrm{olap}}$, and $H_{\mathrm{conn}}$ is made independent and all the constraints are likely to be satisfied. In other words, when the disclosed technique is used, a plurality of constraint conditions are likely to be satisfied at the same time. As a result, when the disclosed technique is used, it is possible to efficiently search for a stable structure.

(Structure Search Method and Structure Search Apparatus)

The structure search method disclosed herein is a method for searching for a stable structure of a compound in which n compound groups are coupled.

The structure search method is a method using a computer.

The structure search method includes a process of creating a three-dimensional structure and a process of calculating the minimum energy, and further includes another process according to a demand.

The structure search apparatus disclosed herein includes a unit that creates a three-dimensional structure and a unit that calculates the minimum energy, and further includes another unit according to a demand.

The structure search apparatus includes, for example, a memory and a processor, and further includes another unit according to a demand.

The processor is coupled to the memory.

The processor is configured to perform the process of creating a three-dimensional structure.

The processor is configured to perform the process of calculating the minimum energy.

The processor is, for example, a central processing unit (CPU), a graphics processing unit (GPU), or a combination thereof.

In the process of creating a three-dimensional structure, n compound groups are sequentially arranged at each lattice point of a three-dimensional lattice space, which is a set of lattices, to create a three-dimensional structure of a compound in the three-dimensional lattice space.

The unit for creating a three-dimensional structure sequentially arranges the n compound groups at each lattice point of a three-dimensional lattice space, which is a set of lattices, and creates a three-dimensional structure of a compound in the three-dimensional lattice space.

The compound group is, for example, an amino acid residue.

In a case where the compound group is an amino acid residue, examples of the compound include a protein.

An amino acid to be the origin of the amino acid residue may be a natural amino acid or an artificial amino acid. Examples of the natural amino acid include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, β-alanine, β-phenylalanine, and the like. Examples of the artificial amino acid include parabenzoyl phenylalanine and the like.

The number of amino acid residues in the protein is not particularly limited and may be appropriately selected according to the purpose, and for example, may be about 10 to 30, or may be several hundreds.

For example, as long as the protein is a protein targeted for medium-molecule drug discovery, the number may be about 10 to 30.

Next, in the process of calculating the minimum energy, the ground state search using the annealing method is performed on the Ising model converted based on the constraint condition for each lattice point, thereby calculating the minimum energy of the Ising model.

The unit for calculating the minimum energy performs the ground state search using the annealing method on the Ising model converted based on the constraint condition for each lattice point, thereby calculating the minimum energy of the Ising model.

The constraint conditions include a first constraint, a second constraint, and a third constraint.

The first constraint is that each of the n compound groups is arranged at only one lattice point.

The second constraint is that the n compound groups do not overlap with each other at each lattice point.

The third constraint is a constraint relating to coupling of n compound groups, and is a constraint that increases the energy of the Ising model calculated when the constraint is not satisfied.

The third constraint is, for example, a constraint expressed by the following (1) and (2).

(1) In a case where the compound group is present at a certain lattice point, the compound group is present at only one lattice point among all lattice points adjacent to the lattice point.

(2) In a case where no compound group is present at a certain lattice point, no compound group is present at all lattice points adjacent to the lattice point, or the compound group is present at only one lattice point among all the lattice points adjacent to the lattice point.

An example of the third constraint may be expressed by the following Formula (E). This example is an example of a two-dimensional case using the diamond encoding method.

$$H \mathrel{+}= C(Q - q_0)(Q - 1)$$
$$Q = \Sigma_{i \in \eta(q_0)} q_i = q_1 + q_2 + q_3 + q_4$$

Formula (E)

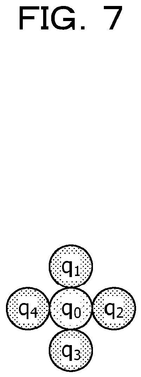

In the Formula, C is a coefficient for weighting and is a positive integer. Each of $q_0$, $q_1$, $q_2$, $q_3$, and $q_4$ takes "1" or "0". A positional relationship of $q_0$, $q_1$, $q_2$, $q_3$, and $q_4$ is the positional relationship illustrated in FIG. 7.

$\eta(q_0)$ is a set of bits representing a compound group adjacent to and coupled to $q_0$.

A case where $q_0$ is "1" is a case where the compound group is present at a certain lattice point. In a case where $q_0$ is "1", H is "0" only when Q is "1". In a case of the positional relationship illustrated in FIG. 7, Q is "1" when $q_1 + q_2 + q_3 + q_4 = 1$. In other words, it is a case where only one of $q_1$, $q_2$, $q_3$, and $q_4$ is "1". Therefore, H is "0" in a case where the compound group is present at only one lattice point among all the lattice points adjacent to a certain lattice point.

A case where $q_0$ is "0" means a case where no compound group is present at a certain lattice point. In a case where $q_0$ is "0", H is "0" when Q is "0" or when Q is "1". In the case of the positional relationship illustrated in FIG. 7, H is "0" in a case where $q_1 + q_2 + q_3 + q_4 = 0$ or 1. In other words, H is "0" in a case where all of $q_1$, $q_2$, $q_3$, and $q_4$ are "0" or a case where only one of $q_1$, $q_2$, $q_3$, and $q_4$ is "1". Therefore, H is "0" in a case where no compound group is present at any of the lattice points adjacent to a certain lattice point or in a case where the compound group is present at only one lattice point among all the lattice points adjacent to the certain lattice point.

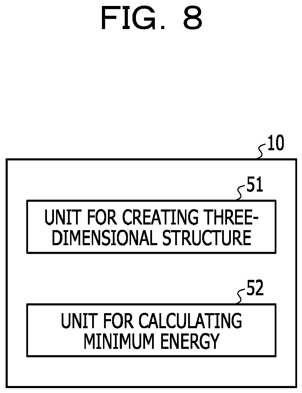

FIG. 8 illustrates a block diagram of an example of the disclosed structure search apparatus.

A structure search apparatus 10 of FIG. 8 includes a unit 51 for creating a three-dimensional structure and a unit 52 for calculating the minimum energy.

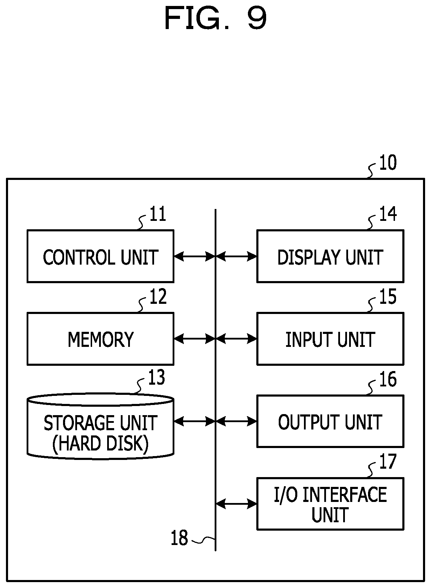

FIG. 9 illustrates a configuration example of the disclosed structure search apparatus.

The structure search apparatus 10 is configured, for example, such that a control unit 11, a memory 12, a storage unit 13, a display unit 14, an input unit 15, an output unit 16, an I/O interface unit 17, and the like are coupled via a system bus 18.

The control unit 11 is a processor that performs arithmetic operations (four arithmetic operations, comparison operation, and the like), operation control of hardware and software, and the like.

The memory 12 is a memory such as a random-access memory (RAM), a read-only memory (ROM), or the like. The RAM stores an operating system (OS), an application program, and the like read out from the ROM and the storage unit 13, and functions as a main memory and a work area of the control unit 11.

The storage unit 13 is a device for storing various programs and data, and is a hard disk, for example. The storage unit 13 stores a program to be executed by the control unit 11, data to be used for execution of the program, the OS, and the like.

The program is stored in the storage unit 13, loaded into the RAM (main memory) of the memory 12, and executed by the control unit 11.

The display unit 14 is a display device, and is, for example, a display apparatus such as a CRT monitor, a liquid crystal panel, or the like.

The input unit 15 is an input device for various data, and is, for example, a keyboard, a pointing device (for example, a mouse, or the like), or the like.

The output unit 16 is an output device for various data, and is, for example, a printer.

The I/O interface unit 17 is an interface for coupling various external devices. For example, the I/O interface unit 17 allows input and output of data of a compact disc read-only memory (CD-ROM), a digital versatile disk read-only memory (DVD-ROM), a magneto-optical (MO) disk, a Universal Serial Bus (USB) memory, or the like.

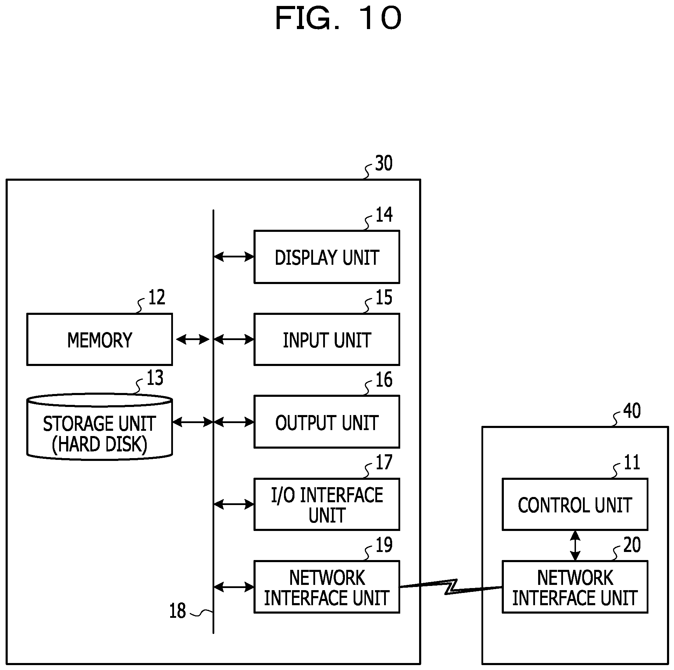

FIG. 10 illustrates another configuration example of the disclosed structure search apparatus.

The configuration example of FIG. 10 is a cloud-type configuration example and includes elements 11-18 described with reference to FIG. 9, and the control unit 11 is independent from the storage unit 13 and the like. In the configuration example, a computer 30 that stores the storage unit 13 and the like, and a computer 40 that stores the control unit 11 are coupled via network interface units 19 and 20.

The network interface units 19 and 20 are hardware that performs communication using the Internet.

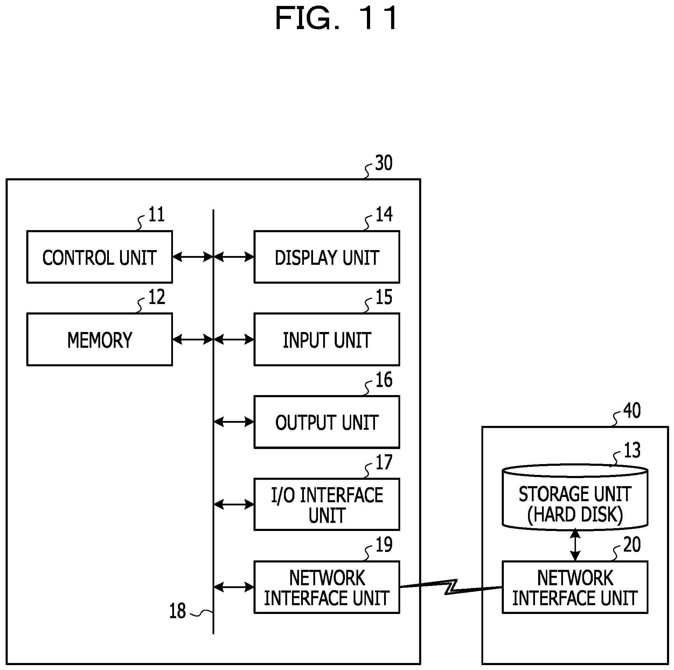

FIG. 11 illustrates another configuration example of the disclosed structure search apparatus.

The configuration example of FIG. 11 is a cloud-type configuration example and includes elements 11-18 described with reference to FIG. 9, and the storage unit 13 is independent from the control unit 11 and the like. In the configuration example, the computer 30 that stores the control unit 11 and the like, and the computer 40 that stores the storage unit 13 are coupled via the network interface units 19 and 20.

Hereinafter, an example of the disclosed technique will be described with reference to a flowchart and the like.

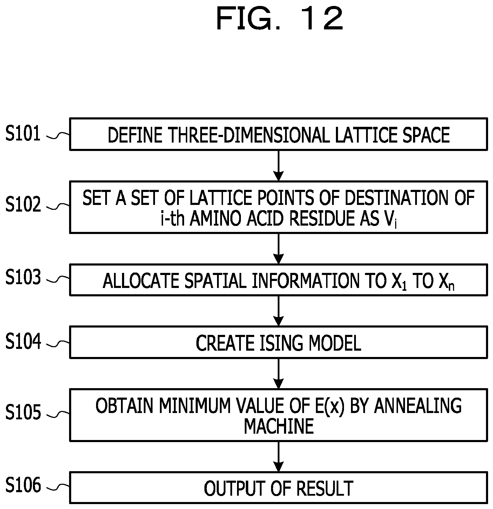

FIG. 12 illustrates a flowchart for searching for a stable structure of a protein.

<Step S101>

First, a three-dimensional lattice space that is a set of lattices in which a plurality of amino acid residues is sequentially arranged is defined based on the number (n) of amino acid residues (S101).

An example of the definition of the three-dimensional lattice space will now be described. The lattice space is three-dimensional, but in the following, a two-dimensional case is described for simplification.

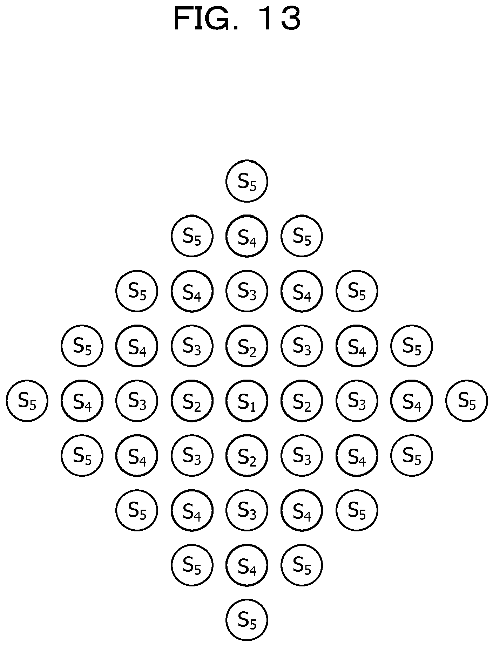

First, a set of lattices having a radius r in a diamond lattice space is referred to as a Shell, and each lattice point is denoted as $S_r$. Each lattice point $S_r$ may be represented as illustrated in FIG. 13.

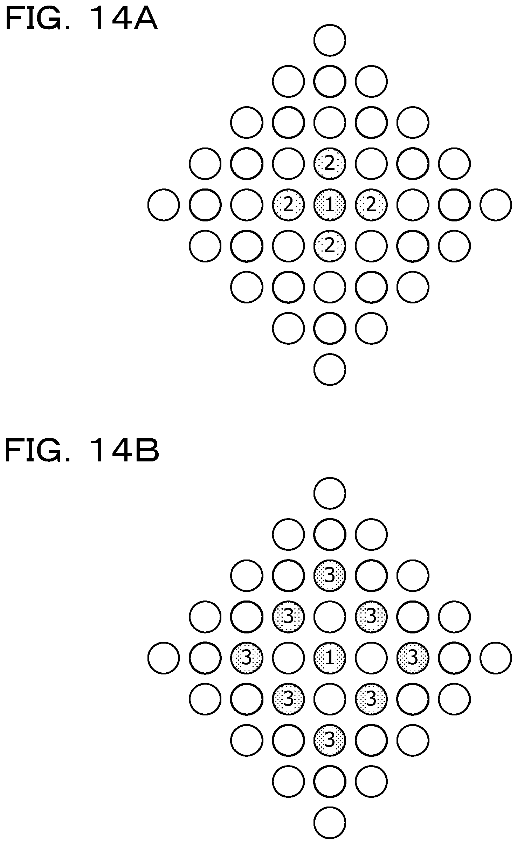

For example, sets $V_1$ to $V_5$ of the lattice points of destinations of first to fifth amino acid residues are as illustrated in FIG. 14A to FIG. 14D. In FIG. 14A to FIG. 14D, a character V of $V_1$ to $V_5$ is omitted, and only the numerical subscripts are displayed.

In FIG. 14A, $V_1 = S_1$, and $V_2 = S_2$.

In FIG. 14B, $V_3 = S_3$.

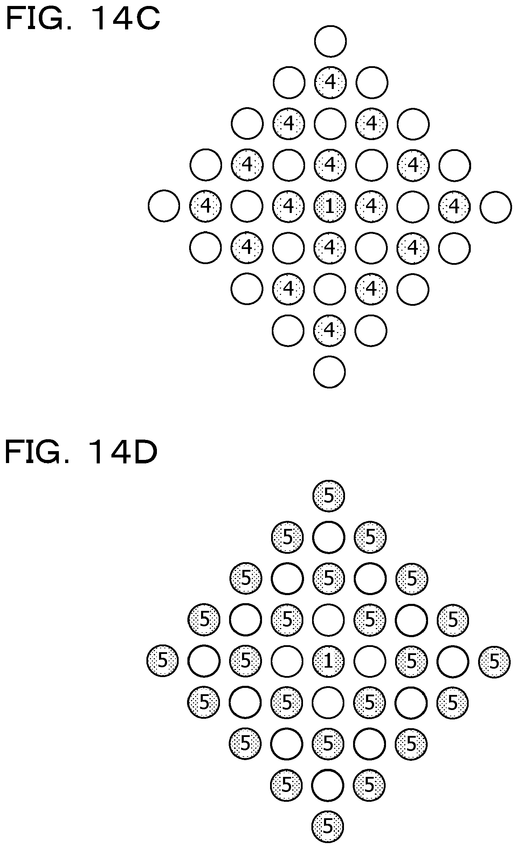

In FIG. 14C, $V_4 = S_2$ or $S_4$.

In FIG. 14D, $V_5 = S_3$ or $S_5$.

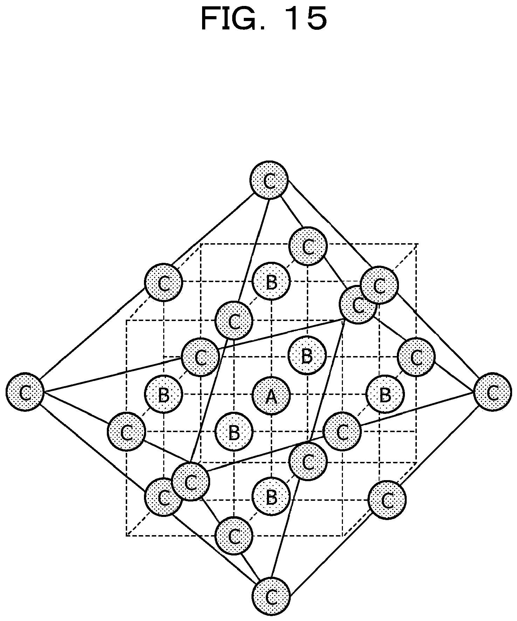

$S_1$, $S_2$, and $S_3$ are expressed in three dimensions as illustrated in FIG. 15. In FIG. 15, $A = S_1$, $B = S_2$, and $C = S_3$.

A space $V_i$ demanded by the i-th amino acid residue in the protein having n amino acid residues is represented by the following equation.

$$V_i = \bigcup_{r \in J} S_r$$

i = {1, 2, 3, ...... n}.

In a case of an odd-numbered (i = odd) amino acid residue, J = {1, 3, ..... i}, and in a case of even-numbered (i = even) amino acid residues, J = {2, 4, ..... i}.

<Step S102>

Next, a set of lattice points of destinations of the i-th amino acid residue is set as $V_i$ (S102).

A space into which an amino acid residue enters is defined.

<Step S103>

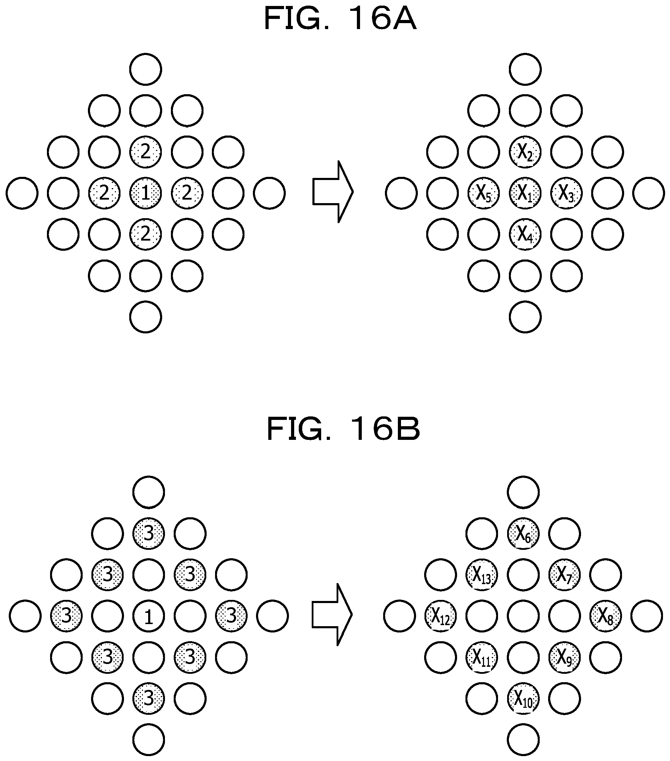

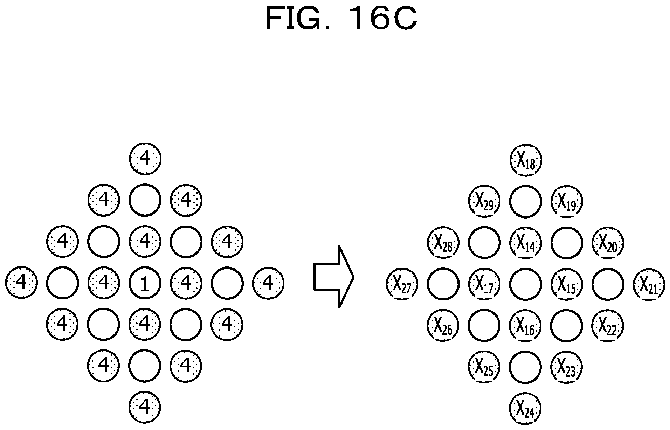

Next, a bit is assigned to each lattice point. In other words, spatial information is allocated to each of bits $X_1$ to $X_n$ (S103). Specifically, as illustrated in FIG. 16A to FIG. 16C, the bits are allocated to the space into which each amino acid residue enters, the bits being represented by "1" with the presence of the amino acid residue at that position and represented by "0" with no presence thereof, respectively. In FIG. 16A to FIG. 16C, a plurality of $X_i$ is assigned to respective amino acid residues 2 to 4, but in practice one bit $X_i$ is assigned to one amino acid residue.

<Step S104>

Next, $H_{one}$, $H_{olap}$, $H_{conn}$, and $H_{pair}$ are set to create the Ising model that is converted based on the constraint condition for each lattice point (S104).

In the diamond encoding method, the total energy may be expressed as follows.

$$E(x) = H = H_{one} + H_{olap} + H_{conn} + H_{pair}$$

$H_{one}$ represents the constraint that the protein includes only one instance of each of the first to n-th amino acid residues (first constraint).

$H_{olap}$ represents the constraint that the first to n-th amino acid residues do not overlap with each other (second constraint).

$H_{conn}$ represents the third constraint.

$H_{pair}$ is the cost function representing the interaction between amino acids.

Examples of $H_{one}$, $H_{olap}$, and $H_{pair}$ are as follows.

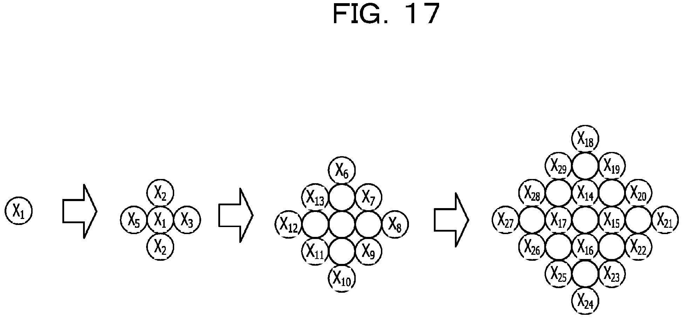

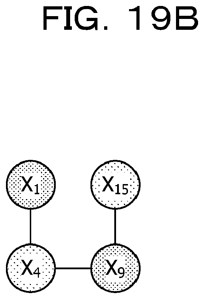

In FIG. 17 to FIG. 19A and FIG. 19B described below, $X_1$ represents a position at which the amino acid residue having the number 1 may be arranged.

$X_2$ to $X_5$ represent positions at which the amino acid residue having the number 2 may be arranged.

$X_6$ to $X_{13}$ represent positions at which the amino acid residue having the number 3 may be arranged.

$X_{14}$ to $X_{29}$ represent positions at which the amino acid residue having the number 4 may be arranged.

An example of $H_{one}$ is described below.

$$H_{one} = \lambda_{one} \sum_{i=0}^{N-1} \sum_{x_a, x_b \in Q_i, a<b} x_a x_b$$

In the above function, $X_a$ and $X_b$ take "1" or "0". In other words, in FIG. 17, $H_{one}$ is a function in which only one of $X_2$, $X_3$, $X_4$, and $X_5$ is "1", so energy is increased in a case where any two or more of them are "1", and a term of a penalty that $H_{one}$ is 0 in a case where only one of $X_2$, $X_3$, $X_4$, and $X_5$ is "1".

In the above function, $\lambda_{one}$ is a coefficient for weighting.

An example of $H_{olap}$ is described below.

$$H_{olap} = \lambda_{olap} \sum_{v \in V} \sum_{x_a, x_b \in \theta(v), a<b} x_a x_b$$

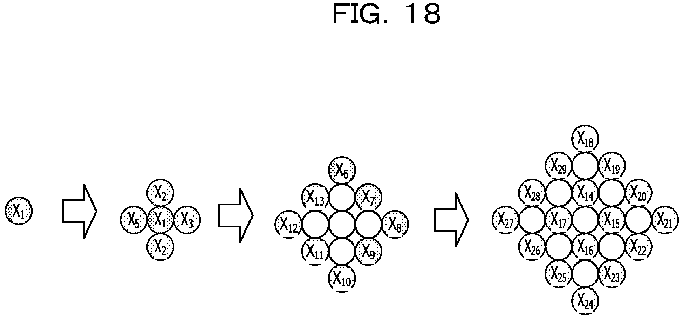

In the above function, $X_a$ and $X_b$ take "1" or "0". In other words, in FIG. 18, $H_{olap}$ is a term where a penalty is generated in a case where $X_{14}$ is "1" when $X_2$ is "1".

In the above function, $\lambda_{olap}$ is a coefficient for weighting.

An example of $H_{pair}$ is described below.

$$H_{pair} = \frac{1}{2} \sum_{i=0}^{N-1} \sum_{x_a \in Q_i} \sum_{x_b \in \eta(x_a)} P_{\omega(x_a)\omega(x_b)} x_a x_b$$

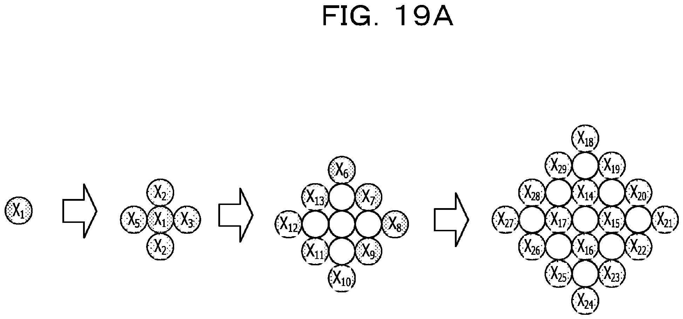

In the above function, $X_a$ and $X_b$ take "1" or "0". In other words, in FIG. 19A and FIG. 19B, $H_{pair}$ is a function in which an interaction $P_{\omega(x1)\omega(x15)}$ acts between the amino acid residue at $X_1$ and the amino acid residue at $X_{15}$ to cause the energy decrease when $X_1$ is "1" in a case where $X_{15}$ is "1". The interaction $P_{\omega(x1)\omega(x15)}$ is determined by the combination of two amino acid residues, and the interaction $P_{\omega(x1)\omega(x15)}$ is determined with reference to, for example, the Miyazawa-Jernigan (MJ) matrix or the like.

The third constraint ($H_{conn}$) is the constraint on the coupling of n compound groups, and is the constraint that increases the energy of the Ising model calculated when the constraint is not satisfied.

The first and second constraints increase the total energy when the respective constraints are not satisfied. In other words, the first constraint and the second constraint are constraints that destabilize the structure of a protein when the respective constraints are not satisfied.

The total energy is increased when the third constraint is not satisfied. The third constraint is a constraint that the structure of a protein is destabilized when the third constraint is not satisfied.

In a case where the third constraint is a constraint that reduces the total energy when the constraint is not satisfied, the relationship between the first constraint and the second constraint, and the third constraint is non-independent, and when one constraint is satisfied, another constraint is less likely to be satisfied. As a result, it is difficult to efficiently search for a stable structure.

However, in the disclosed technique, the third constraint is the constraint that increases the total energy when the constraint is not satisfied. Therefore, the relationship between the first constraint and the second constraint, and the third constraint becomes independent, whereby all the constraints are likely to be satisfied. In other words, it is possible to efficiently search for a stable structure by simultaneously satisfying a plurality of constraint conditions.

The third constraint is, for example, the constraint represented by the following (1) and (2).

(1) In a case where the compound group is present at a certain lattice point, the compound group is present at only one lattice point among all lattice points adjacent to the lattice point.

(2) In a case where no compound group is present at a certain lattice point, no compound group is present at all lattice points adjacent to the lattice point, or the compound group is present at only one lattice point among all the lattice points adjacent to the lattice point.

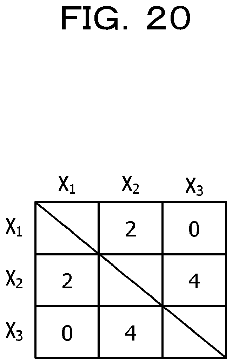

Next, a weight file that is extracted and optimized through the calculation using the energy equation of the following Ising model and corresponds to a weight coefficient (for example, $\lambda_{one}$, $\lambda_{olap}$, $\lambda_{conn}$, $\lambda_{pair}$, or the like) in the respective above functions is, for example, a matrix, and is a file of the matrix as illustrated in FIG. 20 in a case of $2X_1X_2 + 4X_2X_3$.

By using the created weight file, it is possible to express the following energy equation of the Ising model.

$$E(x) = -\sum_{(i,j)} W_{ij} x_i x_j - \sum_i b_i x_i$$

In the above function, states $X_i$ and $X_j$ are "0" or "1", and "0" means that no amino acid residue is present, and "1" means that the amino acid residue is present. $W_{ij}$ in a first term on the right side is a coefficient for weighting.

The first term on the right side represents the sum of products of states of two circuits and a weight value without missing or redundantly counting for all combinations of two circuits selectable from all circuits.

A second term on the right side represents the sum of products of individual bias values and the state of all the circuits. $b_i$ represents a bias value of an i-th circuit.

<Step S105>

Next, in an annealing machine, the ground state search using the annealing method is performed on the Ising model converted based on the constraint condition for each lattice point, thereby calculating the minimum energy of the Ising model (S105).

The annealing machine is not particularly limited as long as it is a computer employing an annealing method for performing the ground state search on an energy function expressed by the Ising model, and may be appropriately selected depending on the purpose. Examples of the annealing machine include, for example, a quantum annealing machine, a semiconductor annealing machine using a semiconductor technique, a machine for performing simulated annealing to be executed by software using a CPU or a graphics processing unit (GPU), and the like. As the annealing machine, for example, a Digital Annealer (registered trademark) may be used.

An example of the annealing method and the annealing machine will be described below.

The annealing method is a method of stochastically obtaining a solution by using a random number value or a superposition of quantum bits. Hereinafter, a problem of minimizing a value of an evaluation function to be optimized will be described as an example, and the value of the evaluation function will be referred to as energy. In a case where the value of the evaluation function is maximized, a sign of the evaluation function may be changed.

First, starting with an initial state in which one discrete value is assigned to each variable, based on a current state (a combination of values of variables), a state close to the current state (for example, a state in which only one of the variables has been changed) is selected, and this state transition is examined. A change in energy associated with the state transition is calculated, and according to the calculated value it is stochastically determined whether to adopt the state transition and change the current state or to maintain the original state without adopting the state transition. When setting an adoption probability of a state transition that results in a drop in the energy to be larger than that of a state transition that results in a rise in the energy, state changes occur in a direction in which the energy drops on average, and thus it is possible to expect that the state is transitioned to a more suitable state with the lapse of time. Therefore, there is a possibility that an optimal solution or an approximate solution that results in energy close to an optimal value may be finally obtained.

When a state transition that results in a drop in the energy is adopted and a state transition that results in a rise in the energy is not adopted in a deterministic way, the change in energy broadly monotonically decreases over time, but once a local solution is reached, no further change may occur. Since an extraordinarily large number of local solutions is present in a discrete optimization problem as described above, the state is stuck at the local solution that is not very close to the optimal value, in many cases. Therefore, in solving a discrete optimization problem, it is important to determine whether or not to adopt the state stochastically.

In the annealing method, it has been proven that the state reaches the optimal solution at a limit of infinite time (the number of iterations) when the adoption (acceptance) probability of the state transition is determined as follows.

Hereinafter, a method for determining an optimal solution using the annealing method will be described in order.

(1) For an energy change (energy decrease) value ($-\Delta E$) associated with a state transition, an acceptance probability p of the state transition is determined by any of the following functions f( ).

$$p(\Delta E, T) = f(-\Delta E / T) \qquad \text{(Formula 1-1)}$$

$$f_{metro}(x) = \min(1, e^{x}) \qquad \text{(Metropolis method)(Formula 1-2)}$$

$$f_{Gibbs}(x) = \frac{1}{1 + e^{-x}} \qquad \text{(Gibbs method)(Formula 1-3)}$$

T is a parameter called a temperature value, and for example, may be changed as follows.

(2) The temperature value T is logarithmically reduced with respect to the number of iterations t as expressed by the following equation.

$$T = \frac{T_0 \log(c)}{\log(t + c)} \qquad \text{(Formula 2)}$$

$T_0$ represents an initial temperature value and it is desirable that a sufficiently large value be set in accordance with the problem.

In a case where the acceptance probability expressed by Formula (1) is used, when a steady state is reached after the sufficient number of iterations, an occupation probability of each state follows a Boltzmann distribution for a thermal equilibrium state in thermodynamics.

Since the occupation probability of a lower-energy state increases when the temperature is gradually decreased from the high initial temperature, the low-energy state is supposed to be obtained when the temperature sufficiently decreases. This method is referred to as the annealing method (or simulated annealing method) because this performance resembles a state change in annealing a material. The stochastic occurrence of the state transition that results in a rise in the energy corresponds to thermal excitation in physics.

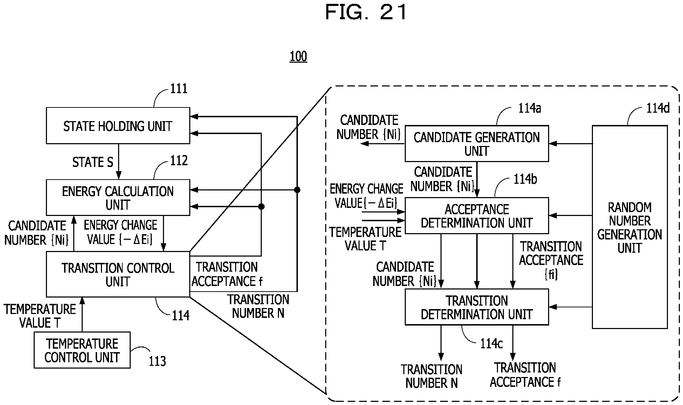

FIG. 21 illustrates an example of a functional configuration of an optimization apparatus that performs the annealing method. While a case where a plurality of candidates for the state transition is generated will be also described in the following description, the transition candidates are generated one by one in the basic annealing method.

An optimization apparatus 100 includes a state holding unit 111 that holds a current state S (values of a plurality of state variables). The optimization apparatus 100 also includes an energy calculation unit 112 that calculates energy change values {-ΔEi} of the respective state transitions in a case where the state transition occurs from the current state S as a result of change in any of the values of the plurality of state variables. The optimization apparatus 100 further includes a temperature control unit 113 that controls a temperature value T and a transition control unit 114 that controls state changes.

The transition control unit 114 stochastically determines whether or not any one of a plurality of state transitions is accepted, depending on a relative relationship between the energy change values {-ΔEi} and thermal excitation energy based on the temperature value T, the energy change values {-ΔEi}, and the random number value.

The transition control unit 114 includes a candidate generation unit 114a for generating candidates for a state transition, and an acceptance determination unit 114b for stochastically determining whether or not the state transition is allowed for each candidate from the energy change values {-ΔEi} of the candidate and the temperature value T. The transition control unit 114 includes a transition determination unit 114c for determining a candidate to be adopted from the accepted candidates, and a random number generation unit 114d for generating a probability variable.

The operation in one iteration in the optimization apparatus 100 is as follows.

First, the candidate generation unit 114a generates one or a plurality of candidates (candidate numbers {Ni}) for the state transition from the current state S held by the state holding unit 111 to the next state. Next, the energy calculation unit 112 calculates the energy change values {-ΔEi} for each of the state transitions listed as a candidate, by using the current state S and the candidates for the state transition. The acceptance determination unit 114b accepts the state transition with the acceptance probability obtained by the above Formula (1) using the temperature value T generated in the temperature control unit 113 and the probability variable (random number value) generated by the random number generation unit 114d according to the energy change values {-ΔEi} of each of the state transitions.

The acceptance determination unit 114b outputs acceptances {fi} of the respective state transitions. In a case where a plurality of state transitions is accepted, the transition determination unit 114c randomly selects one of them by using the random number value. The transition determination unit 114c then outputs a transition number N of the selected state transition, and a transition acceptance f. In a case where an accepted state transition is present, the value of the state variable stored in the state holding unit 111 is updated according to the adopted state transition.

Starting with the initial state, the above iteration processes are repeated while causing the temperature control unit 113 to lower the temperature value, and the operation ends when a certain number of iterations is reached, or when an end determination condition, such as a condition that the energy becomes lower than a predetermined value, is satisfied. A solution output by the optimization apparatus 100 is the state at the operation end.

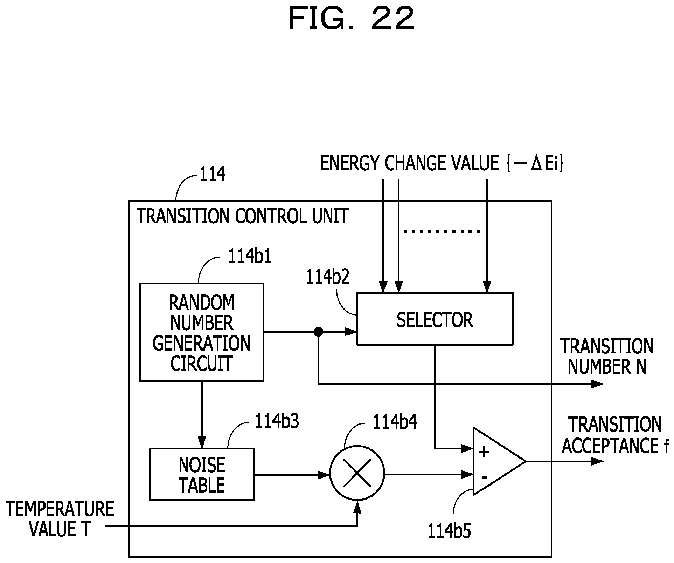

FIG. 22 is a circuit level block diagram of a configuration example of the transition control unit in a normal annealing method for generating candidates one by one, especially, an arithmetic portion demanded for the acceptance determination unit.

The transition control unit 114 includes a random number generation circuit 114b1, a selector 114b2, a noise table 114b3, a multiplier 114b4, and a comparator 114b5.

Of the energy change values {-ΔEi} calculated for the candidates of the respective state transitions, the selector 114b2 selects and outputs an energy change value corresponding to the transition number N, which is a random number value generated by the random number generation circuit 114b1.

Functions of the noise table 114b3 will be described later. As the noise table 114b3, for example, a memory such as a RAM, a flash memory, or the like may be used.

The multiplier 114b4 outputs a product (corresponding to the thermal excitation energy described above) obtained by multiplying a value output from the noise table 114b3 by the temperature value T.

The comparator 114b5 outputs a comparison result as the transition acceptance f obtained by comparing a multiplication result output by the multiplier 114b4 with the energy change value -ΔE selected by the selector 114b2.

While the transition control unit 114 illustrated in FIG. 22 basically implements the functions described above as they are, a mechanism of accepting the state transition with the acceptance probability expressed by Formula (1) will be described in more detail.

It is possible to realize a circuit that outputs 1 and 0 with the acceptance probabilities p and (1-p), respectively, by a comparator that has two inputs A and B, outputting 1 when A > B, outputting 0 when A < B, in a manner such that the acceptance probability p is input to the input A and a uniform random number taking values in an interval [0, 1) is input to the input B. Thus, by inputting the value of the acceptance probability p calculated by using Formula (1) based on the energy change value and the temperature value T to the input A of the comparator, it is possible to achieve the above function.

In other words, when it is assumed that f is the function used in Formula (1) and u is the uniform random number taking values in the interval [0, 1), it is possible to achieve the above function by the circuit that outputs 1 when f(ΔE/T) is larger than u.

It is possible achieve the same function as that described above with the following change.

Even when the same monotonically increasing function is allowed to act on two numbers, the two numbers maintain the same magnitude relationship. Therefore, even when the same monotonically increasing function is allowed to act on the two inputs of the comparator, the same output is obtained. When an inverse function $f^{-1}$ of f is adopted as this monotonically increasing function, it is seen that a circuit that outputs 1 when $-\Delta E/T$ is larger than $f^{-1}(u)$ may be provided. Since the temperature value T is positive, it is seen that a circuit that outputs 1 when $-\Delta E$ is larger than $Tf^{-1}(u)$ is suitable.

The noise table 114b3 in FIG. 22 is a conversion table for achieving the inverse function $f^{-1}(u)$, and is a table for outputting a value of the following function with respect to the input obtained by discretizing the interval [0, 1).

$$f_{metro}^{-1}(u) = \log(u)$$  (Formula 3-1)

$$f_{Gibbs}^{-1}(u) = \log\left(\frac{u}{1-u}\right)$$  (Formula 3-2)

Although the transition control unit 114 is provided with a latch that holds a determination result and the like, a state machine that generates the corresponding timing, and the like, they are not illustrated in FIG. 22 in order to simplify the illustration.

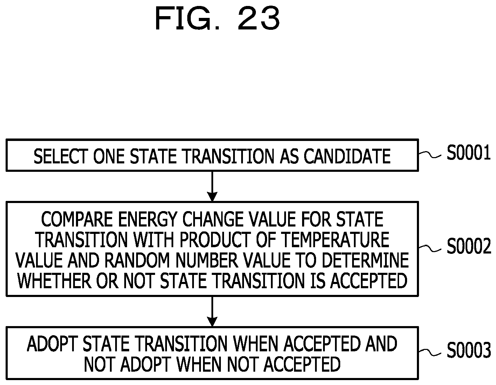

FIG. 23 is a diagram illustrating an example of an operation flow of the transition control unit 114. The operation flow illustrated in FIG. 23 includes a step of selecting one state transition as a candidate (S0001), a step of comparing the energy change value for the state transition with a product of a temperature value and a random number value to determine whether or not a state transition is accepted (S0002), and a step of adopting the state transition when the state transition is accepted and not adopting the state transition when the state transition is not accepted (S0003).

<Step S106>

In S106, a calculation result is output. The result may be output as a three-dimensional structure diagram of a protein or as coordinate information of each amino acid residue constituting a protein.

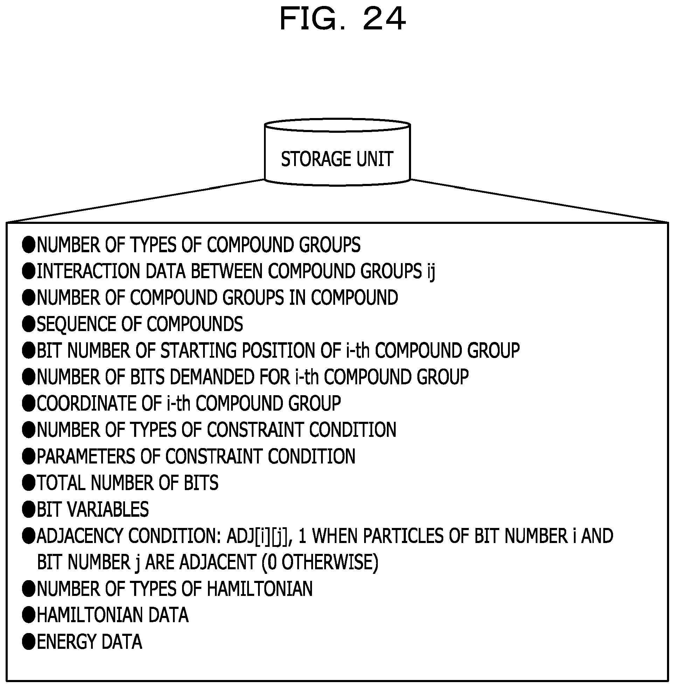

FIG. 24 illustrates a data configuration example of the storage unit of the structure search apparatus.

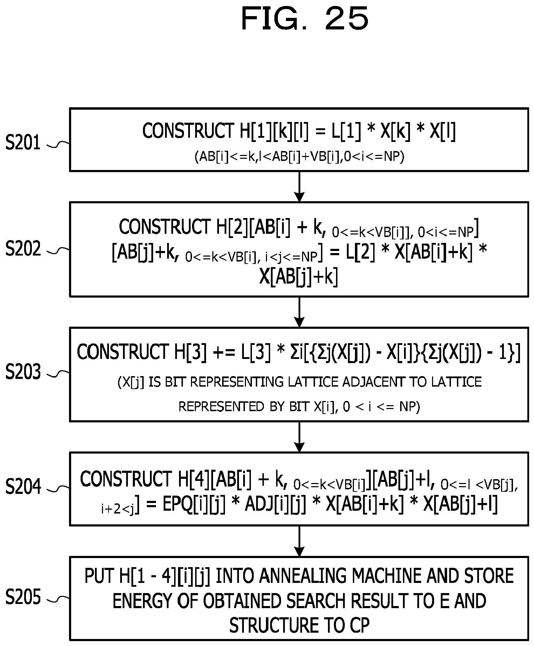

FIG. 25 illustrates a processing flow corresponding to the data configuration example.

In the processing flow illustrated in FIG. 25, first, in S201, a first constraint is constructed. The first constraint here is expressed as follows.

<First Constraint>

$$H[1][k][l] = L[1]*X[k]*X[l]$$

Subsequently, in S202, a second constraint is constructed. The second constraint here is expressed as follows.

<Second Constraint>

$$H[2][AB[i]+k, \; 0<=k<VB[i]], \; 0<i<=NP][AB[j]+k, \; 0<=k<VB[i], \; i<j<=NP]$$
$$= L[2]*X[AB[i]+k]*X[AB[j]+k]$$

Subsequently, in S203, a third constraint is constructed. The third constraint here is expressed as follows.

<Third Constraint>

$$H[3] += L[3] * \Sigma_i [\{\Sigma_j(X[j]) - X[i]\}\{\Sigma_j(X[j]) - 1\}]$$

Subsequently, in S204, a cost function based on the interaction between two adjacent compound groups is constructed. The cost function here is expressed as follows.

<Cost Function>

$$H[4][AB[i] + k,\ _{0<=k<VB[i]}][AB[j] + l,\ _{0<=l<VB[j],i+2<j}]$$
$$= EPQ[i][j] * ADJ[i][j] * X[AB[i] + k] * X[AB[j] + l]$$

Subsequently, in S205, the constructed first constraint, second constraint, third constraint, and cost function are used to perform the ground state search using the annealing method on the Ising model transformed based on the constraint condition for each lattice point in the annealing machine. The obtained energy is stored in E of the storage unit, and the obtained structure is stored in the storage unit.

With respect to the third constraint in the disclosed structure search method, Hamiltonian ($H_{conn}$) in the related art is expressed as follows, for example.

$$H[3][AB[i] + k,\ _{0<=k<VB[i],\ 0<=i<NP}][AB[i+1] + l,\ _{0<=l<VB[i+1],\ 0<=i<NP}]$$
$$= -L[3] * \Sigma(ADJ(AB[i] + k, AB[i+1] + l) * X[AB[i] + k] * X[AB[i+1] + l])$$

(Program)

The disclosed structure search program is a program that causes a computer to execute the disclosed structure search method.

In the structure search program, an aspect in execution of the structure search method is the same as an aspect in the disclosed structure search method.

The program may be created using various known program languages according to a configuration of a computer system to be used, and a type, a version, and the like of an operating system.

The program may be recorded on a recording medium such as an internal hard disk or an external hard disk, or may be recorded on a recording medium such as a compact disc read-only memory (CD-ROM), a digital versatile disk read-only memory (DVD-ROM), a magneto-optical (MO) disk, or a Universal Serial Bus (USB) memory (USB flash drive). In a case where the program is recorded on the recording medium such as the CD-ROM, the DVD-ROM, the MO disk, the USB memory, or the like, the program may be used directly through a recording medium reading device included in the computer system or may be used by being installed on the hard disk according to the purpose. The program may be recorded in an external storage area (another computer or the like) accessible from the computer system through the information communication network, and also may be used directly through the information communication network from the external storage area or may be used by being installed on the hard disk according to the purpose.

The program may be recorded in a plurality of recording media by being divided for each arbitrary processing.

(Recording Medium)

The disclosed recording medium records the disclosed structure search program.

The disclosed recording medium is computer-readable.

The disclosed recording medium may be transitory or non-transitory.

The disclosed recording medium is, for example, a recording medium that records a program for causing a computer to execute the disclosed structure search method.

The recording medium is not particularly limited, and may be appropriately selected according to the purpose, and examples thereof include, for example, an internal hard disk, an external hard disk, a CD-ROM, a DVD-ROM, an MO disk, a USB memory, and the like.

The recording medium may be a plurality of recording media in which the program is divided and recorded for each arbitrary processing.

[Experimental Example]

Hereinafter, specific experimental examples of the present embodiment will be described.

(Comparative Example 1)

The stable structure search of the coarse-grained lattice model of Chignolin protein was performed according to the flowchart of FIG. 12.

$H_{one}$, $H_{olap}$, $H_{conn}$ were set as the constraint conditions, and $H_{pair}$ was set as the cost function. The total energy in the diamond encoding method may be expressed as follows.

$$E(x) = H = H_{one} + H_{olap} + H_{conn} + H_{pair}$$

$H_{one}$ represents a constraint that the protein includes only one instance of each of the first to n-th amino acid residues.

$H_{olap}$ represents a constraint that the first to n-th amino acid residues do not overlap with each other.

$H_{conn}$ is a constraint that the first to n-th amino acid residues are linked to each other.

$H_{pair}$ is the cost function representing the interaction between the amino acid residues.

Among 216 available patterns of three parameters that set the constraint conditions (in a case where each parameter takes a value of an integer multiple of 5 from 5 to 30), only two patterns were able to search for the most stable structure.

For each of the two patterns, the search for 300,000 iterations of annealing was performed 20 times using an annealing machine, and as a result of obtaining the minimum value of $E(x)$, the most stable structure was reached only one time.

(Experimental Example 1)

The stable structure search of the coarse-grained lattice model of Chignolin protein was performed according to the flowchart of FIG. 12 in the same manner as in Comparative Example 1 except that $H_{conn}$ in Comparative Example 1 was replaced with the third constraint of the disclosed technique.

The third constraint is a constraint expressed by the following (1) and (2).

(1A) In a case where the amino acid residue is present at a certain lattice point, the amino acid residue is present at only one lattice point among all lattice points adjacent to the lattice point.

(2) In a case where no amino acid residue is present at a certain lattice point, no amino acid residue is present at any lattice points adjacent to the lattice point, or the amino acid residue is present at only one lattice point among all lattice points adjacent to the lattice point.

All of 216 available patterns of three parameters that set the constraint conditions (in a case where each parameter takes a value of an integer multiple of 5 from 5 to 30) were able to search for the most stable structure.

For each of the 216 patterns, the search for 300,000 iterations of annealing was performed 20 times using an annealing machine, and as a result of obtaining the minimum value of $E(x)$, all the patterns reached the most stable structure.

From the comparison between Comparative Example 1 and Experimental Example 1, it was confirmed that the disclosed technique was able to efficiently search for a stable structure.

In any of the above aspects, the various features may be implemented in hardware, or as software modules running on one or more processors/computers.

The invention also provides a computer program or a computer program product comprising instructions which, when executed by a computer, cause the computer to carry out any of the methods/method steps described herein, and a non-transitory computer-readable medium comprising instructions which, when executed by a computer, cause the computer to carry out any of the methods/method steps described herein. A computer program embodying the invention may be stored on a non-transitory computer-readable medium, or it could, for example, be in the form of a signal such as a downloadable data signal provided from an Internet website, or it could be in any other form.

**Claims**

1. A structure search method, comprising:

   sequentially arranging, by a computer, n compound groups at each lattice point, of a plurality of lattice points, of a three-dimensional lattice space to create a three-dimensional structure of a compound in the three-dimensional lattice space, the n compound groups being coupled to each other in the compound; and
   calculating a minimum energy of an Ising model by performing a ground state search using an annealing method on the Ising model transformed based on constraint conditions for each of the lattice points, the constraint conditions including:

      a first constraint that each of the n compound groups is arranged at only one lattice point;
      a second constraint that the n compound groups do not overlap with each other at each of the lattice points; and
      a third constraint that relates to coupling of the n compound groups and increases energy of the Ising model calculated when the constraint is not satisfied.

2. The structure search method according to claim 1, wherein
   the third constraint is a constraint that
   in a case where a compound group is present at a first lattice point, a compound group is present only at one lattice point among all lattice points adjacent to the first lattice point, and
   in a case where no compound group is present at the first lattice point, no compound group is present at any lattice points adjacent to the first lattice point, or a compound group is present at only one lattice point among all lattice points adjacent to the first lattice point.

3. The structure search method according to claim 1 or 2, wherein
   the compound is a protein, and
   the compound group is an amino acid residue.

4. A program that causes a computer to execute a process, the process comprising:

   sequentially arranging n compound groups at each lattice point, of a plurality of lattice points, of a three-dimensional lattice space to create a three-dimensional structure of a compound in the three-dimensional lattice space, the n compound groups being coupled to each other in the compound; and
   calculating a minimum energy of an Ising model by performing a ground state search using an annealing method on the Ising model transformed based on constraint conditions for each of the lattice points, the constraint conditions including:

      a first constraint that each of the n compound groups is arranged at only one lattice point;
      a second constraint that the n compound groups do not overlap with each other at each of the lattice points; and
      a third constraint that relates to coupling of the n compound groups and increases energy of the Ising model calculated when the constraint is not satisfied.

5. The program according to claim 4, wherein
   the third constraint is a constraint that
   in a case where a compound group is present at a first lattice point, a compound group is present only at one lattice point among all lattice points adjacent to the first lattice point, and
   in a case where no compound group is present at the first lattice point, no compound group is present at any lattice points adjacent to the first lattice point, or a compound group is present at only one lattice point among all lattice points adjacent to the first lattice point.

6. The program according to claim 4 or 5, wherein
   the compound is a protein, and
   the compound group is an amino acid residue.

7. A structure search apparatus, comprising:

a unit that sequentially arranges n compound groups at each lattice point, of a plurality of lattice points, of a three-dimensional lattice space to create a three-dimensional structure of a compound in the three-dimensional lattice space, the n compound groups being coupled to each other in the compound; and

a unit that calculates a minimum energy of an Ising model by performing a ground state search using an annealing method on the Ising model transformed based on constraint conditions for each of the lattice points, the constraint conditions including:

a first constraint that each of the n compound groups is arranged at only one lattice point;

a second constraint that the n compound groups do not overlap with each other at each of the lattice points; and

a third constraint that relates to coupling of the n compound groups and increases energy of the Ising model calculated when the constraint is not satisfied.

8. The structure search apparatus according to claim 7, wherein
the third constraint is a constraint that
in a case where a compound group is present at a first lattice point, a compound group is present only at one lattice point among all lattice points adjacent to the first lattice point, and
in a case where no compound group is present at the first lattice point, no compound group is present at any lattice points adjacent to the first lattice point, or a compound group is present at only one lattice point among all lattice points adjacent to the first lattice point.

9. The structure search apparatus according to claim 7 or 8, wherein
the compound is a protein, and
the compound group is an amino acid residue.

10. A computer readable storage medium having the program of any of claims 4 to 6 stored thereon.

FIG. 1A

FIG. 1B

FIG. 1C

FIG. 2A

FIG. 2B

FIG. 2C

FIG. 2D

FIG. 2E

# FIG. 3

# FIG. 4

# FIG. 5

# FIG. 6

# FIG. 7

# FIG. 8

10

51

| UNIT FOR CREATING THREE-DIMENSIONAL STRUCTURE |

52

| UNIT FOR CALCULATING MINIMUM ENERGY |

# FIG. 9

# FIG. 10

# FIG. 11

# FIG. 12

S101 — DEFINE THREE-DIMENSIONAL LATTICE SPACE

S102 — SET A SET OF LATTICE POINTS OF DESTINATION OF i-th AMINO ACID RESIDUE AS $V_i$

S103 — ALLOCATE SPATIAL INFORMATION TO $X_1$ TO $X_n$

S104 — CREATE ISING MODEL

S105 — OBTAIN MINIMUM VALUE OF E(x) BY ANNEALING MACHINE

S106 — OUTPUT OF RESULT

# FIG. 13

FIG. 14A

FIG. 14B

## FIG. 14C

## FIG. 14D

FIG. 15

## FIG. 16A

## FIG. 16B

# FIG. 16C

FIG. 17

# FIG. 18

# FIG. 19A

# FIG. 19B

# FIG. 20

|       | X₁  | X₂  | X₃  |
|-------|-----|-----|-----|
| X₁    |     | 2   | 0   |
| X₂    | 2   |     | 4   |
| X₃    | 0   | 4   |     |

FIG. 21

# FIG. 22

# FIG. 23

SELECT ONE STATE TRANSITION AS CANDIDATE — S0001

COMPARE ENERGY CHANGE VALUE FOR STATE TRANSITION WITH PRODUCT OF TEMPERATURE VALUE AND RANDOM NUMBER VALUE TO DETERMINE WHETHER OR NOT STATE TRANSITION IS ACCEPTED — S0002

ADOPT STATE TRANSITION WHEN ACCEPTED AND NOT ADOPT WHEN NOT ACCEPTED — S0003

# FIG. 24

STORAGE UNIT

- NUMBER OF TYPES OF COMPOUND GROUPS
- INTERACTION DATA BETWEEN COMPOUND GROUPS ij
- NUMBER OF COMPOUND GROUPS IN COMPOUND
- SEQUENCE OF COMPOUNDS
- BIT NUMBER OF STARTING POSITION OF i-th COMPOUND GROUP
- NUMBER OF BITS DEMANDED FOR i-th COMPOUND GROUP
- COORDINATE OF i-th COMPOUND GROUP
- NUMBER OF TYPES OF CONSTRAINT CONDITION
- PARAMETERS OF CONSTRAINT CONDITION
- TOTAL NUMBER OF BITS
- BIT VARIABLES
- ADJACENCY CONDITION: ADJ[i][j], 1 WHEN PARTICLES OF BIT NUMBER i AND BIT NUMBER j ARE ADJACENT (0 OTHERWISE)
- NUMBER OF TYPES OF HAMILTONIAN
- HAMILTONIAN DATA
- ENERGY DATA

# FIG. 25

S201 — CONSTRUCT H[1][k][l] = L[1] * X[k] * X[l]
(AB[i]<=k,l<AB[i]+VB[i],0<i<=NP)

S202 — CONSTRUCT H[2][AB[i] + k, $_{0<=k<VB[i]]}$, 0<i<=NP]
[AB[j]+k, $_{0<=k<VB[i], i<j<=NP}$] = L[2] * X[AB[i]+k] * X[AB[j]+k]

S203 — CONSTRUCT H[3] += L[3] * Σi[{Σj(X[j]) - X[i]}{Σj(X[j]) - 1}]
(X[j] IS BIT REPRESENTING LATTICE ADJACENT TO LATTICE
REPRESENTED BY BIT X[i], 0 < i <= NP)

S204 — CONSTRUCT H[4][AB[i] + k, $_{0<=k<VB[i]}$][AB[j]+l, $_{0<=l<VB[j]}$, $_{i+2<j}$] = EPQ[i][j] * ADJ[i][j] * X[AB[i]+k] * X[AB[j]+l]

S205 — PUT H[1 - 4][i][j] INTO ANNEALING MACHINE AND STORE
ENERGY OF OBTAINED SEARCH RESULT TO E AND
STRUCTURE TO CP

Europäisches
Patentamt

European
Patent Office

Office européen
des brevets

# EUROPEAN SEARCH REPORT

**Application Number**

EP 20 20 2456

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Ryan Babbush ET AL: "Construction of Energy Functions for Lattice Heteropolymer Models: Efficient Encodings for Constraint Satisfaction Programming and Quantum Annealing" In: "Advances in Chemical Physics: Volume 155", 11 April 2014 (2014-04-11), John Wiley & Sons, Inc., Hoboken, New Jersey, XP055692281, ISBN: 978-1-118-75577-8 pages 201-244, DOI: 10.1002/9781118755815.ch05, * page 221 - page 224 * * page 232 - page 239 * ----- | 1-10 | INV. G16C20/40 G16B15/00 |
| X | TOMAS BABEJ ET AL: "Coarse-grained lattice protein folding on a quantum annealer", ARXIV.ORG, CORNELL UNIVERSITY LIBRARY, 201 OLIN LIBRARY CORNELL UNIVERSITY ITHACA, NY 14853, 2 November 2018 (2018-11-02), XP080941413, * the whole document * ----- | 1-10 | |
| | | | TECHNICAL FIELDS SEARCHED (IPC) |
| X | KANAMARU SHO ET AL: "Mapping Constrained Slot-Placement Problems to Ising Models and its Evaluations by an Ising Machine", 2019 IEEE 9TH INTERNATIONAL CONFERENCE ON CONSUMER ELECTRONICS (ICCE-BERLIN), IEEE, 8 September 2019 (2019-09-08), pages 221-226, XP033694054, DOI: 10.1109/ICCE-BERLIN47944.2019.8966207 [retrieved on 2020-01-21] * the whole document * ----- | 1-10 | G16C G16B |

The present search report has been drawn up for all claims

| Place of search | Date of completion of the search | Examiner |
|---|---|---|
| The Hague | 4 March 2021 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **R. BABBUSH.** Construction of Energy Functions for Lattice Heteropolymer Models: A Case Study in Constraint Satisfaction Programming and Adiabatic Quantum Optimization. *Advances in Chemical Physics,* 04 April 2014, vol. 155, 201-244